(19) Europäisches Patentamt
European Patent Office
Office européen des brevets

(11) Veröffentlichungsnummer : **0 053 319**
**B1**

(12) # EUROPÄISCHE PATENTSCHRIFT

(45) Veröffentlichungstag der Patentschrift : **09.01.85**

(21) Anmeldenummer : **81109726.0**

(22) Anmeldetag : **17.11.81**

(51) Int. Cl.⁴ : **C 07 C154/02**, C 07 D317/24, C 07 D319/06, C 08 F 2/38, C 08 F136/18

(54) **Verfahren zur Herstellung von Xanthogendisulfiden und deren Verwendung als Molgewichtsregler bei der Polymerisation von Chloropren.**

(30) Priorität : **28.11.80 DE 3044811**

(43) Veröffentlichungstag der Anmeldung : **09.06.82 Patentblatt 82/23**

(45) Bekanntmachung des Hinweises auf die Patenterteilung : **09.01.85 Patentblatt 85/02**

(84) Benannte Vertragsstaaten : **BE DE FR GB IT NL**

(56) Entgegenhaltungen :
**DE-A- 2 306 610**
**DE-B- 1 162 833**
**DE-C- 853 445**
**US-A- 1 491 021**
**US-A- 2 270 257**
**US-H- 901 006**

(73) Patentinhaber : **BAYER AG**
**Konzernverwaltung RP Patentabteilung**
**D-5090 Leverkusen 1 Bayerwerk (DE)**

(72) Erfinder : **Arend, Günter, Dr.**
**Claudiusstrasse 42**
**D-4047 Dormagen (DE)**
Erfinder : **Königshofen, Heinrich, Dr.**
**Am Mühlenberg 26**
**D-5060 Bergisch-Gladbach 2 (DE)**
Erfinder : **Müller, Peter, Dr.**
**Ahornstrasse 2**
**D-5014 Kerpen (DE)**
Erfinder : **Musch, Rüdiger, Dr.**
**Altenburger Dom-Strasse 169**
**D-5060 Bergisch-Gladbach 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft die Herstellung von Xanthogendisulfiden mit einem Gehalt an elementaren Schwefel von unter 1,7 Gew.-%, durch Oxidation von Xanthogensäure-O-ester-alkalisalzen mit Chlorbleichlauge, sowie ihre Verwendung als Molgewichtsregler bei der Polymerisation von Chloropren zu hellen Polymerisaten.

Xanthogendisulfide sind wichtige Molekulargewichtsregler bei der Polymerisation von ungesättigten Monomeren, insbesondere von Chloropren. Die Produkte müssen von guter Reinheit und in hoher Ausbeute herstellbar sein. Verfahren zur Herstellung von Xanthogendisulfiden sind in der Literatur mehrfach beschrieben. Durch Umsetzung von Alkolen mit Schwenfelkohlenstoff in Gegenwart von mindestens äquimolaren Mengen Alkali stellt man Xanthogensäure-O-estersalze her, welche oxidativ zu Xanthogendisulfiden umgesetzt werden können. Anfänglich wurden Jod oder kupfersulfat als Oxidationsmittel verwendet (Zeise : Schw. J. 1822, *36 ;* Berz. J. 1824, *3,* 82 ; 1837, *16,* 306 ; Ann. Phys. 1835, *35,* 489 sowie « Houben-Weyl, Meth. der Organ. Chemie » ; Bd. 9, S. 812 (1955)), später wurden als Oxidationsmittel Natriumtetrathionat, Bromcyan, Salpetrige Säure, Chloramin-T, Nitrosylchlorid (siehe : Cambron, Whitby : Canadian J. Res., 1930, *2,* 144) sowie ein elektrolytisches Verfahren (Schall : Z. Elektrochem. 1895, *2,* 475) beschrieben. In DE-OS 2 306 610 sind weiterhin als Oxidationsmittel Kaliumperoxodisulfat oder Wasserstoffperoxid bekannt. Für eine großtechnische Verwendung sind diese Oxidationsmittel jedoch zu teuer und auch sicherheitstechnisch nicht unbedenklich. In der US-Patentschrift 2 375 083 und in der DE-OS 2 533 989 wird Chlor- bzw. Bromdampf, gegebenenfalls in Verdünnung mit Luft, als Oxidationsmittel vorgeschlagen.

Alle diese Prozesse ergeben im Regelfall gute Ausbeuten.

Die erhaltenen Reinheiten sind jedoch ohne Zwischenschaltung eines Reinigungsschrittes für den praktischen Einsatz dieser Disulfide als Polymerisationsregler nicht ausreichend, obwohl die Verbindungen elementaranalytisch rein zu sein scheinen. In der Kanadischen Patenschrift 856 834 wird daher vorgeschlagen, die Oxidation der Xanthogenate mit Chlorbleichlauge im wäßrigen System in Gegenwart eines niederen aliphatischen Alkohols, insbesondere Isopropanols durchzuführen. Durch die Mitverwendung des Alkohols werden Produkte von heller Farbe und kleinem Schmelzpunktintervall erhalten.

Nach DE-B-1 162 833 wird mit verhältnismäßig konzentrierten Natriumhypochlorit-Lösungen oxidiert und die Oxidation bis zum Verbrauch des Xanthogenats durchgeführt. Die Produkte enthalten relativ viel elementaren Schwefel.

Die im rein wäßrigen System durch Chlor-, Chlorbleichlauge-, Chloramin-T- oder Persulfatoxidation hergestellten Xanthogendisulfide lassen sich gegebenenfalls durch mehrfache Destillation oder Umkristallisation so rein erhalten, daß ihr Einsatz als Molekulargewichtsregler möglich wird. Diese Reinigungsoperationen sind technisch jedoch nur aufwendig realisierbar. So kann man Diethylxanthogendisulfid bei 0,05 mm Druck destillieren, bei 18 mm zersetzt sich die Verbindung bereits quantitativ bei der versuchten Destillation (Tschugaeff-Reaktion, siehe hierzu : Bulmer, Mann : J. Chem. Soc. *1945,* 674 ff.). Viele Xanthogendisulfide lassen sich umkristallisieren, wegen ihrer leichten Zersetzlichkeit bei thermischer Belastung kann man jedoch nur geringe Temperaturunterschiede zur Umkristallisierung nutzen : daher ist auch diese Reiningungsmethode problematisch und führt zu übermäßigen Ausbeuteverlusten. Zusätzlich ist eine Regenerierung der verwendeten Lösungsmittel erforderlich, welche wegen der leichten thermischen Zersetzlichkeit der gelösten Xanthogenatreste (Tschugaeff-Reaktion : Mercaptanbildung !) ebanfalls nur unter besondere Vorkehrungen technisch möglich ist. Ein Verfahren, welches Xanthogendisulfide mit billigen Oxidationsmitteln im wäßrigen Medium in sehr sauberer Form herzustellen gestattet, wäre somit ein bedeutender technischer Fortschritt.

Um die Reinheit der Xanthogendisulfide zu ermitteln, wurde außer den üblichen physikalischen Daten ihre Eignung als Molekulargewichtsregler bei der Chloroprenpolymerisation getestet. Dabei wurde Chloropren in einem Standardansatz unter Zusatz fester Mengen von Xanthogendisulfid plymerisiert, das resultierende Elastomer wurde aufgearbeitet und die Mooney-Viskosität bestimmt (siehe Beispiel 1a).

Im Falle des Bis-(5-ethyl-1,3-dioxan-5-yl)-methyl-xanthogendisulfids (abgekürzt « MTX ») erbringen gute Produktqualitäten eine Mooney-Viskosität (ML-4'-Wert) von maximal 50, im Durchschnittels 35 bis 45 ; bei schlechten Partien wurde teilweise ML-4' > 100 gefunden. Von allen untersuchten Spezifikationsgrößen des Reglers konnte eine Korreletion zwischen Mooney-Viskosität des Elastomeren im Standardansatz und dem HPLC-analytisch gefundenem Schwefelgehalt im MTX festgstellt werden (zur HPLC-Analytik des MTX siehe Beispiel 1 b). Wegen des geringen absoluten Schwefelgehaltes im Xanthogendisulfid ist die Analyse naturgemäß mit einem relativ großen Fehler behaftet, so daß die Korrelation statistisch untermauert wurde. In Schaubild 1 ist die Abhängigkeit des ML-4'-Wertes vom Schwefelgehalt aufgetragen, jeder Punkt entspricht dem Ergebnis eines Polymerisationsversuchs von Chloropren mit jeweils wechselnden Versuchspartien an MTX. Man sieht, daß die Spezifikationsobergrenze von ML-4' = 50 regelmäßig überschritten wird, wenn der Schwefelgehalt im Regler über 1,5 bis 1,7 % beträgt. Die beobachtete und in der Patentliteratur (z. B. Kanadische Patenschrift 856 834) erwähnte mangelhafte Reglerwirkung von in rein wäßrigen Medium hergestellten Xanthogendisulfiden ist somit auf einen Gehalt an Elementarschwefel zurückzuführen.

Dieser Schwefelgehalt ist weder durch eine Verfälschung der Elementaranalysenergebnisse noch durch eine Schmelzpunktsdepression nachweisbar.

Der höhere Schwefelgehalt schlechterer Reglerpartien läßt sich auch mit dem Verbrauch an Aktivatorlösung bei der Polymerisation von Chloropren korrelieren (vgl. Beispiel 1 und Tabelle 1). Polymere, welche unter Verwendung von MTX mit einem hohen Schwefelgehalt hergestellt wurden, zeigen bei der Heißluftlagerung (3 Tage bei 70 °C) einen starken Anstieg des Mooney-Wertes.

Die Erfindung betrifft somit ein Verfahren zur Herstellung von Xanthogendisulfiden der Formel

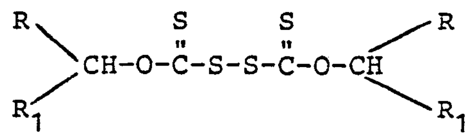

(I)

in der

R und $R_1$ unabhängig voneinander Wasserstoff, $C_1$-$C_6$-Alkyl, $C_5$-$C_8$-Cycloalkyl oder ein sauerstoffhaltiges, gegebenenfalls 1- bis 3-fach durch $C_1$-$C_4$-Alkyl substituiertes, heterocyclisches Ringsystem mit 5-8 Ringgliedern darstellen, wobei

R und $R_1$ darüber hinaus auch miteinander zu einem heterocyclischen Ring mit 3-6 C-Atomen und 1-3 Sauerstoffringgliedern verbunden sein können, der gegebenenfalls durch 1-3 $C_1$-$C_4$-Alkylgruppen substituiert sein kann,

durch Umsetzung eines Xanthogensäure-O-ester-alkalisalzes der Formel

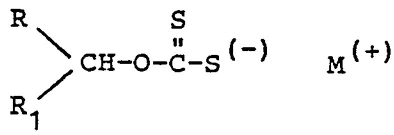

(II)

in der

R und $R_1$ die vorstehend genannte Bedeutung besitzen und

M für ein Alkalimetall, bevorzugt für Natrium oder Kalium steht,

mit wäßriger Chlorbleichlauge, bei einer Temperatur von − 10 bis 20 °C, vorzugsweise 0 bis 10 °C und bei einem pH-Wert von 8 bis 12, vorzugsweise 8 bis 11, dadurch gekennzeichnet, daß man wäßrige Chlorbleichlauge einer Konzentration von weniger als 0,9 Mol NaOCl/kg, vorzugsweise 0,4 bis 0,7 Mol/kg einsetzt, und nur so weit oxidiert, daß zum Ende der Reaktion das Xanthogensäure-O-ester-alkalisalz zu 0,1 bis 5 Mol-%, vorzugsweise zu 1 bis 3 Mol-%, bezogen auf die eingesetzte Mengen, noch vorhanden ist.

Die erfindungsgemäß hergestellten Xanthogendisulfide haben Gehalte an elementarem Schwefel von unter 1,7 Gew.-%, vorzugsweise unter 1,2 Gew.-%.

Bevorzugt stellt R Wasserstoff und $R_1$ 5-Ethyl-1,3-dioxan-5-yl oder 1,3-Dioxolan-4-yl dar.

Der erfindungsgemäße Endpunkt der Umsetzung kann leicht jodometrisch bestimmt werden. Das gebildete Xanthogendisulfid wird nach bekannten Verfahren isoliert, wie beispielsweise als Flüssigphase abgetrennt oder gegebenenfalls als Feststoff abfiltriert und getrocknet.

Die Oxidation ist empfindlich gegenüber Abweichungen von den genannten Reaktionsbedingungen, insbesondere gegen die Verwendung von höherkonzentrierter Chlorbleichlauge, das Arbeiten bei zu hoher Temperatur, zu niedrigem pH-Wert sowie einer Überoxidation, die auch leicht lokal durch suchlechte Durchmischung eintreten kann. Solche Abweichungen führen bei großtechnischen Ansätzen zu Xanthogendisulfiden mit erhöhtem Gehalt an freiem Schwefel.

Das Xanthogesaüresalz der Formel II kann getrennt oder in situ hergestellt werden.

In einer bevorzugten Durchführungsform des erfindungsgemäßen Verfahrens stellt man in einem separaten Verfahrensschritt nach bekannten Vorschriften das Xanthogensäureestersalz der allgemeinen Formel II in reiner Form her, siehe hierzu biespielsweise : « Houben-Weyl : Methoden der Organischen Chemie ; Bd. 9, S. 812 (1955) ».

Sollte eine in situ-Herstellung in Betracht kommen, stellt man in einer besonders bevorzugten Form des erfindungsgemäßen Verfahrens aus einer Mischung des betreffenden Alkohols in 0 bis 20 Mol-%, vorzugsweise 3 bis 15 Mol-% Überschuß, auf $CS_2$ mit Schwefelkohlenstoff durch Zutropfen von vorzugsweise 50 Gew.-%iger Natronlauge das Xanthogenat her und setzt es direkt ohne Zwischenisolierung weiter um. Bei der in situ-Herstellung des Xanthogenats kann gegebenenfalls zur Vermeidung von Wanthogenkristallen mit Wasser verdünnt werden, jedoch sollte das Molverhältnis Schwefeskohlenstoff zu Wasser kleiner als 1 : 20, vorzugsweise kleiner als 1 : 10 sein, da sonst leicht Hydrolyse unter Bildung von Natriumsulfid eintreten kann. Aus dem gleichen Grund sollte bei einer Temperatur unterhalb von 40 °C, vorzugsweise bei 0 bis 20 °C, gearbeitet werden. Die Xanthogenatbildung ist nach maximal 12 Stunden beendet, die Reaktion kann leicht jodometrisch verfolgt werden.

Zu dem Xanthogenat bzw. zu dessen konzentrierter wäßriger Lösung gibt man unter sehr guter Durchmischung die verdünnte Chlorbleichlauge so rasch hinzu, wie es die Kühlung des Reaktors erlaubt und hält gleichzeitig den pH-Wert durch Zugabe einer Säure wie beispielsweise Schwefelsäure,

Saulzsäure, Salpetersäure, Phosphorsäure oder Essigsäure im gewünschten Bereich. Die Synthese wird am besten durch die jodometrisch kontrollierte Abnahme des Xanthogengehaltes analytisch verfolgt. Da Xanthogendisulfide durch Chlorbleichlauge in langsamer Reaktion unter anderem unter Bildung von Schwefel oxidativ angegriffen werden, bricht man die Synthese bei einem geringen Restgehalt an Xanthogenat vorzeitig ab, der Restgehalt sollte zwischen 0,1 und 5 Mol-%, vorzugsweise zwischen 1 und 3 Mol-%, bezogen auf den Einsatz, betragen.

Trägt man den Verbrauch an Chlorbleichlauge gegen den Xanthogenatumsatz graphisch auf, so findet man strenge Proportionaltät bis etwa 95 % Umsatz. Von da an steigt der Chlorbleichlaugebverbrauch überproportional (siehe Beispiel 4e sowie Schaubild 2). Die allgemein übliche Endpunkbestimmung der Oxidationsreaktion mit Kaliumjodid/Stärkepapier kann hier also, insbesondere bei großen Ansätzen, wo noch Durchmischungsprobleme vorhanden sind, nicht angewendet werden.

Um eine ausreichend gute Einmischung der Chlorbleichlauge in die Reaktionsmischung zu erreichen, muß diese bei großen Ansätzen direkt an der Eintrittsstelle fein verteilt werden. So hat sich z. B. das Einsprühen der Chlorlauge in die Flüssigphase oder in den Gasraum des Oxidationskessels bewährt. Besonders bevorzugt wird ein Verfahren, bei dem man den Kesselinhalt über einen externen kreislauf umpumpt, wobei die Chlorbleichlauge mittels einer gewöhnlichen Mischdüse (Injektionsdüse) im Seitenstrom zugeführt wird.

Da im alkalischen Reaktionsmilieu die Chlorbleichlauge durch Disproportionierung von Chlor hergestellt werden kann, ist es möglich, die erfindungsgemäße Synthese auch mit Chlor anstelle von Chlorbleichlauge durchzuführen. Um Überoxidation des Xanthogendisulfids zu vermeiden, muß die Reaktion mit einem stark durch Luft oder Stickstofff verdünnten Chlorstrom durchgeführt werden, insbesondere ein Volumenverhältnis Chlor/Inertgas von 1 zu 20 bis 1 zu 100 hat sich hier bewährt.

Verwendet man höher konzentrierte Chlorbleichlauge, so ist ebenfalls mit einer ötlichen Überoxidation zu rechnen. Bei besonders lagsamen Zulauf und besonders guter Durchmischung ist der Einsatz solcher Chlorbleichlauge grundsätzlich möglich, geht jedoch auf Kosten einer längeren Reaktionszeit und hoher Prozeßunsicherheit. Es ist möglich, den Oxidationsprozed sowohl diskontinuerlich im Rührwerksreaktor wie kontinuierlich z. B. in einer Reaktorenkaskade, einem Rohr- bzw. Schangenreaktor oder einem Schneckenapparat durchzuführen.

Die diskontinuierliche Fahrweise hat sich im Hinblick auf die Endpunkteinstellung als vorteilhaft erweisen, während im Konti-Prozeß eine bessere Durchmischung und einfachere Temperierung erzielbar ist. Die Enstcheidung für eine dieser prozeßerten hängt daher nur von den jeweiligen betrieblichen Gegebenheiten ab.

Die Erfindung betrifft weiterhin die Verwendung der erfindungsgemäß hergestellten Xanthogendisulfide als Molgewichtsregler bei der Polymerisation von Chloropren zur Herstellung von benzollöslichen Polymeren. Die Xanthogendisulfide eignen soch besonders für die Darstellung heller Polychloropren-Kautschuke mit sehr guten Vulkanisateigenschaften, deren Verwendung als Klebstoffrohstoff, von Polychloroprenlatices sowie auch in Abmischung mit vernetzten Chloroprenpolymeren des Sol-Gel-Typs gemäß DE-OS 1 720 107.

Die Herstellung von Polychloropren ist seit langem bekannt und beispielsweise in Ullmanns Encyclopädie der technischen Chemie, Stuttgart, Band 9, S. 336 ff., Verlag Urban und Schwarzenberg, München-Berlin 1957 und in Encyclopädia of Polymer Science and Technology, Vol. 3, Seiten 705-730, John Wiley, New-York 1965, beschrieben.

Das Verfahren verläuft zweistufig, wobei in der ersten Stufe die Polymerisation zum Latex durchgeführt wird und in der zweiten Stufe die Aufarbeitung des Latex zum Festkauschuk, beispielsweise durch Gefreirkoagulation erfolgt. Das so herstellte und vulkanisierte Polychloropren besitzt nicht das erwünschte hohe Festigkeitsniveau. Dies läßt sich erreichen, wenn man als Kettenüberträger anstelle von Mercaptan Xanthogendisulfide wie z. B. das Xanthogendisulfid Bis-(5-ethyl-1,3-dioxan-5-yl-methyl) Xanthogen-disulfid (MTX) einsetzt. Das nach dieser Methode hergestellte und als Kettenüberträger eingesetzte Xanthogendisulfid führt einem Polymeren, das nach der Aufarbeitung im Trockner einen mehr oder weniger starken Braunton zeigt. Dies ist auf die merkliche thermische bzw. Strahlenbelastung des Polymeren während des Trockenprozesses zurückzuführen.

Polymere mit einen leichten braunen Eigenfarbe können jedoch beispilsweise zur Herstellung von Mischungen für PKW-Weißwandreifen oder zur Herstellung von Klebstoffen für helle, transparente, verfärbungsfreie Verklebungen nicht eingesetzt werden.

Darüber hinaus besitzen die erfindungsgemäß hergestellten Xanthogendisulfide eine deutlich bessere Reglewirksamkeit und führen daher zu Polychloroprenlatices mit besserer Lagerstabilität. Darüber hinaus benötigt man geringere Aktivatormengen.

Wie bekannt, wird 2-Chlorobutadien (Chloropren) in alkalisch wäßriger Emulsion in Gegenwart von radikalischen Initiatoren polymerisiert. Es ist auch möglich, Cholopren mit verschiedenen Comonomeren zu polymerisieren. Gebräuchliche Comonomere sind z. B. : 1-Chlorbutadien, 2,3-Dichlorbutadien, Styrol, Isopren oder Acrylnitril. Durch Zusatz von MTX, einem Kettennübertragungsmittel, wird das Molekulargewicht des entstandenen Polymeren gesteuert. Die Polymerisationstemperaturen — zwischen 5 und 80 °C möglich — liegen im allgemeinen bei 10-50 °C. Bei diesen Reaktionstemperaturen wird die Polymerisation bei einem Monomerunsatz von 50-85 %, üblicherweise 60-70 %, abgebrochen. Als geeignete Emulgatorsysteme werden Alkalisalze wasserlöslicher gesättigter oder ungesättigter Monocarbonsäuren

eingesetzt, z. B. disproportionierte Resinsäuren, gegebenenfalls im Gemisch mit Fettsäuren wie Ölsäure, Kokosfettsäuren. Die Emulgatoren werden in Mengen von 2-10 Gew.-Teilen (bevorzugt 3-5 Gew.-Teile), bezogen auf 100 Teile Monomer, zugesetzt.

Auch Kondensationsprodukte aus Naphthalinsulfonsäure und Formaldehyd werden als zusätzliche Emulgatoren eingesetzt.

Verwendet man das oben beschriebene Emulgatorsystem, ist ein pH-Wert der Emulsion größer als 10 erforderlich. Es sollte vorzugsweise pH 12-13,5 liegen.

Die Polymerisation wird durch Zugabe von bekannten Polymerisationsinitiatoren gestartet und durchgeführt. Als Initiatoren kommen Radikale erzeugende Verbindungen in Frage, wie z. B. : Alkalipersulfate, Wasserstoffperoxid und organische Peroxide wie Cumolhydroperoxid oder Benzoylperoxid. Es ist ferner möglich, die Polymerisation durch Zugabe von Reduktionsmitteln, wie Formamidinsulfinsäure zu initiieren. Das restliche nicht umgesetzte Monomere kann durch Wasserdampfdestillation entfernt werden. Der pH des alkalischen Latex wird durch verdünnte Essigsäure auf pH 5-7 gesenkt und das Polymere aus dieser Emulsion beispielsweise durch Gefrierkoagulation isoliert und getrocknet, wie z. B. beschrieben in : Engng. Progr. 43, 391 (1974) und in der deutschen Patentschrift 1 051 506. Für die Aufarbeitung eignen sich aber auch andere herkömmliche Methoden, wie z. B. in der deutschen Patenschrift 1 111 804 beschrieben.

Für die Herstellung von Klebstoffen wird das Polychloropren in organischen Lösungsmitteln wie Benzol, Toluol, Methylenchlorid oder Trichlorethylen bzw. in Gemischen dieser Lösungsmittel mit anderen Lösungsmitteln, die Polychloropren allein nicht lösen, wie Benzin, cyclohexan oder Methylacetat, gelöst.

Die Viskosität der Lösung richtet sich nach dem Verwendungszweck und liegt vorzugsweise bei 10-100 Poise, gemessen bei 20 °C mit einem Brookfield-LVT-Viskosimeter.

Weitere Methoden, Polychloroprenklebstoffe herzustellen, werden in der DE-AS 1 200 988 beschrieben.

Die folgenden Beispiele erläutern die Erfindung :

## Beispiel 1

a) Standardansatz einer Chloroprenpolymerisation zur Austestung der Reglewirkung von Bis-(5-ethyl-1,3-dioxan-5-yl-methyl)-xanthogendisulfid (MTX).

Bei 44 °C werden in einem Reaktor vorgelegt (alle Teile sind Gewichtsteile) :

| | |
|---|---|
| 125 | Teile destilliertes Wasser |
| 3,5 | Teile Na-Salz einer disproportionierten Abietinsäure |
| 0,65 | Teile Na-Salz eines Kondensationsproduktes aus Naphthalinsulfonsäure |
| 0,65 | Teile Natriumhydroxid |
| 100 | Teile Chloropren |
| 0,7 | Teile MTX |

Zur Initiierung werden 30 bis 92 Teile einer 2,5 Gew.-%igen Lösung von Formamidinsulfinsäure in Wasser innerhalb von 3,5 Stunden zulaufen lassen. Die Menge an Aktivatorlösung ist abhängig vom Schwefelgehalt des MTX, siehe Tabelle 1.

Bei einem Monomerumsatz von 66 % wird die Reaktion durch Zugabe von Phenothiazin abgebrochen. Das restliche Monomere wird durch Wasserdampfdestillation aus dem Ansatz entfernt. Nach Absenken des pH-Wertes auf 7 fällt man das Polymere mit 0,5 %iger Magnesiumsulfatlösung und dekantiert die wäßrige Phase ab. Man wäscht das Produkt salzfrei und trocknet im Vakuum über Nacht bei 70 °C.

b) HPLC-Bestimmung des Schwefelgehaltes von Bis-(5-ethyl-1,3-dioxan-5-yl-methyl)-xanthogendisulfid (MTX)

Gerät : Liquid-Chromatograph Perkin-Elmer,
Serie 2, UV-Detektor Perkin-Elmer
LC 15
Schreiber
Säule 250 × 4,6 mm
Stationäre Phase : Li-Chromosorb Si 60,5 mm
Mobile Phase : 30 ml n-Butanol
27 ml Acetonitril
mit n-Hexan auf 1 l aufgefüllt
Durchfluß : 1,5ml/Min.
Einspritzmenge : 5 µl
Einspritzlösung : 10 mg MTX-Probe werden auf der Analysenwaage in einem 25 ml-Meßkolben eingewogen und mit mobiler Phase aufgefüllt.

Temperatur : 25 °C
Retentionszeit : Schwefel 2,3 Min.
MTX 4,7 Min.

Die Eichung der Methode erfolgt durch Zuwiegen von Schwefel zu reinstem MTX.
Durch Zuwiegen von Schwefel zu Reinst-MTX hergestellte Proben ergaben folgende Mooney-Viskositäten :

Tabelle 1

| | | | | | | |
|---|---|---|---|---|---|---|
| S-Gehalt des MTX (Gew.-%) | 0 | 0,5 | 1 | 2 | 4 | 8 |
| ML-4'-Wert 100°C | 34 | 36 | 38 | 56 | 73 | 140 |
| Aktivator-Verbrauch bei Polymerisation (Gew.-Teile, siehe Beispiel 1a) | 38 | 43 | 46 | 60 | 78 | 92 |

Eine Reihe von unterschiedlichsten MTX-Proben aus verschiedenen Syntheseversuchen wurden als Regler nach Beispiel 1a eingesetzt und die Mooney-Viskositäten der erhaltenen Elastomeren bestimmt. Das Ergebnis ist in Schaubild 1 graphisch aufgetragen. Die eingezeichnete gestrichelte Gerade entspricht den Werten von Tabelle 1. Schaubild 1 beweist den Zusammenhang von Mooney-Viskosität und Schwefelgehalt des Reglers MT eindeutig. Die Einzelwerte streuen relativ stark, da sowohl die Messung der Mooney-Viskosität einen Fehler von ± 3-5 %, je nach Meßbereich, als auch die Bestimmung geringer Mengen Schwefel nach der HPLC-Analyse einen Fehler bis ca. 20 % aufweisen kann. Der ML-4'-Wert 50 wird stets überschritten, wenn der Schwefelgehalt im MTX größer als 1,7 % ist.

Bei den MTD-Partien mit hohen Schwefelgehalten von über 2 % werden oft überproportional große Mooney-Viskositäten gemessen. Dieser Effekt rührt von anderen unbekannten Verunreinigungen im MTX her, welche durch oxidative Schädigung entstehen. Wahrscheinlich handelt es sich um Polysulfide als Vorstufen der Schwefelabscheidung, welche bei der HPLC-Analyse nicht als Schwefel erfaßt werden, bei der Polymerisation aber wie Schwefel in den Prozeß eingreifen.

In Tabelle 1 ist zusätzlich noch der Verbrauch an Aktivator aufgeführt. Man erkennt, wie der steigende Schwefelgehalt im MTX auch die Polymerisation hemmt, was im kontinuierlich steigenden Aktivatorverbrauch zum Ausdruck kommt. Parallel verschlechtern sich auch polymerphysikalische Eigenschaften, wie beispielsweise die Lagerstabilität in Heißluft, meßbar am Anstieg des Mooney-Wertes um bis zu 25 %.

Beispiel 2

Herstellung von Bis-(5-ethyl-1,3-dioxan-5-yl-methyl)-Xanthogensulfid unter verschiedenen Reaktionsbedingungen :

A) Xanthogenatsynthese
In einem Reaktor werden vorgelegt :

16,33 kg (112 Mol) 5-Ethyl-5-hydroxymethyl-1,3-dioxan
5,8 l (322 Mol) Wasser
7,6 kg (100 Mol) Schwefelkohlenstoff

Bei 10 bis 15 °C werden innerhalb von 5 bis 7 Stunden 8,9 kg (111 Mol) 50 Gew.-%ige Natronlauge zulaufen lassen. Man läßt noch 3 Stunden bei 10 °C nachrühren. Der jodometrisch bestimmte Gehalte an Xanthogenat beträgt 98 %.

$B_1$) Oxidation

Zu der Xanthogenatlösung läßt man in einem 250 l Va-Kessel mit Ankerrührer, (n = 120 $Min.^{-1}$) bei 5 bis 10 °C zunächst 7,6 kg (4,55 Molm Chlorbleichlauge der Konzentration 0,6 Mol/kg, Dichte $_d^{20}$ = 1,97, hinzulaufen. Dann stellt man den pH-Wert durch Zugabe von 20 Gew.-%iger Schwefelsäure oder Salzsäure auf 9 bis 10 ein und läßt dann bei 5 bis 10 °C weiter Chlorbleichlauge der genannten Konzentration zulaufen, wobei man durch parallele Säurezugabe den pH-Wert zwischen 9 und 10 hält. Man stoppt die Zuläufe, wenn der jodometrisch bestimmte Restxanthogenatgehalt noch 1 bis 3 Gew.-% der Ausgangsmenge beträgt, man verbraucht ca. 108 kg (65 Mol) Chlorbleichlauge. Die Suspension wird filtriert, chlorid- frei gewaschen und im Vakuum getrocknet.

Ausbeute : 82 bis 86 %, bezogen auf $CS_2$
Unlöslicher Anteil in Aceton : < 0,1 %
Freier Schwefel nach HPLC : < 0,5 % ; Fp. = 51-53 °C
Das Produkt ist fast weiß, grobkristallin und gut rieselfähig.

$B_2$) Führt man den gleichen Versuch durch, reduziert jedoch bei der Oxidationsstufe die Rührerdrehzahl auf n = 25 $Min.^{-1}$, so erhält man ein schmieriges Produkt, welches sich im Kessel zu kugeligen Gebilden solcher Größe zusammenballt, daß sie nicht über das Bodenventil entnommen werden können. Eine Ausbeutebestimmung ist nicht möglich. Es erweist sich, daß die Durchmischung zu gering war.

| | |
|---|---|
| Farbe : | Dunkelgelb |
| Fp. : | 42-47 °C |
| Freier Schwefelgehalt : | 1,9 % |

Beispiel 3

Beispiel 2 wird wie beschrieben in einer Ansatzgröße von 12,2 kMol, bezogen auf $CS_2$, in einem 23 $m^3$ fassenden Reaktor mit Kreuzbalkenrührer und Stromstörern bei einer Rührerdrehzahl von 65 $Min.^{-1}$ (maximale Drehzahl) wiederholt. Man erhält ein gelbes, schmieriges Produkt, welches die Filterporen verstopft und nur unter größten Schwierigkeiten zu filtrieren ist. Der Filterkuchen verklebt dabei zu einer steinharten Masse.

| | |
|---|---|
| Fp. : | 48-53 °C |
| Schwefelgehalt : | 1,2 % |

Der Versuch wird wiederholt, jedoch pumpt man aus dem Bodenventil einen Teilstrom des Produktes von 20-25 $m^3$/h über ein Zulaufventil am Kesseldeckel um. Der Umpumpstrom dient als treibstrahl einer Mischdüse, in die im Seitenstrom die Chlorlauge eindosiert wird. Unter sonst gleichen Reaktionsbedingungen erhält man ein fast farbloses Produkt von hervorragender Kristallinität und hervorragender Filtrierbarkeit.

| | |
|---|---|
| Fp. : | 51-53 °C |
| Schwefelgehalt : | 0,1 % |
| Ausbeute : | 2,175 kg ≙ 80,7 % |

Beispiel 4

Beispiel 2 wird wiederholt, jedoch werden die in Tabelle 2 jeweils eingetragenen Parameter der Oxidationsstufe verändert. Nur die Beispiele 4a und 4d sind erfindungsgemäß. Diese Produkte sind zwar qualitativ in Ordnung, die Ausbeuten sind jedoch deutlich niedriger, vgl. mit Beispielen 2 und 3. Die Beispiele 4b, 4c und 4e sind nicht erfindungsgemäß und dienen der Verdeutlichung.

(Siehe Tabelle 2 Seite 8 f.)

Tabelle 2

| Beispiel Nr. | T | pH | Restxanthogenat | Beurteilung |
|---|---|---|---|---|
| 4 a | 15 - 20°C | 9 - 10 | 1 - 3 Mol-% | Ausbeute: 65 %<br>S: 0,8 - 1 % |
| 4 b | 35°C | 9 - 10 | 3 Mol-% | Ausbeute: 2 % |
| 4 c | 5 - 10°C | 6 - 7 | Versuch nicht zu Ende geführt, Abspaltung von $CS_2$ | |
| 4 d | 5 - 10°C | 11 - 12 | 2 Mol-% | Ausbeute: 63 % |
| 4 e | 5 - 10°C | 9 - 10 | Zu ca. 5 % überoxidiert | Schmieriges Produkt,<br>S: 1,9 % |

Bei Beispiel 4e wurde in der Nähe des Endpunktes der Xanthogenumsatz als Funktion des NaOCl-Verbrauches aufgetragen. Bis ca. 97 % Umsatz ist die Beziehung streng linear, das restliche Xanthogenat läßt sich dann nur noch unter Verwendung überproportionaler Mengen an Chlorbleichlauge umsetzen, siehe Schaubild 2. Hieraus ist zu entnehmen, daß bei hohem Umsatz, und erst recht bei einer Überoxidation, Nebenreaktionen in den Vordergrund treten, welche im Interesse einer guten Produktqualität unbedingt vermieden werden müssen.

Beispiel 5

Diethylxanthogendisulfid

In einem Rundkolben werden vorgelegt :

51,5 g (1,12 Mol) Ethanol
58 ml (3,22 Mol) Wasser
76 g (1,0 Mol) Schwefelkohlenstoff

Bei 5-10 °C läßt man innerhalb von 6 Stunden 89 g (1,1 Mol) 50 Gew.-%ige Natronlauge zutropfen. Man läßt über Nacht rühren und verdünnt die orangefarbene Lösung mit 60 ml Wasser. Dann tropft man bei 5-7 °C ca. 100 ml einer Chlorbleichlauge der Konzentration 0,62 Mol/l hinzu und stellt anschließend den pH-Wert mit 20 Gew.-%iger Schwefelsäure auf 9. Man oxidiert mit weiterer Chlorbleichlauge bis zu einem jodometrisch bestimmten Xanthogenatumsatz von 97 %. Die untere, organische Phase wird abgetrennt und im Vakuum bei ca. 40 °C entgast.

Ausbeute : 84 g ≙ 76 % auf $CS_2$. Das Produkt kristallisiert beim Stehen.
Fp. : 22-24 °C
Schwefelgehalt : 0,88 %

Beispiel 6

Bis-cyclohexylxanthogendisulfid

112 g (1,12 Mol) Cyclohexanol
58 ml (3,22 Mol) Wasser
76 g (1,0 Mol) Schwefelkohlenstoff
89 g (1,1 Mol) 50 Gew.-%ige Natronlauge

Nach Oxidation mit Chlorbleichlauge bis zu einem jodometrisch bestimmten Xanthogenumsatz von 97 % erhält man ein viskoses gelbes Öl.

Ausbeute : 110 g ≙ 67 % auf $CS_2$
Schwefelgehalt : 0,58 %

Beispiel 7

Kontinuierliche Herstellung von Bis-(5-ethyl-1,3-dioxan-5-yl-methyl)-Xanthogendisulfid.

A) Xanthogenatsynthese
Das Xanthogenat wurde entsprechend Beispiel 2A hergestellt.

B) Oxidation
In einer 2-Kesselkaskade (Füllvolumen 2,5 l/1,3 l) werden kontinuierlich zugeführt

773 g/h (2 Mol/h) Xanthogenat-Lösung
743 ml/h (1,3 Mol/h) Chlorbleichlauge
~ 450 ml/h Schwefelsäure (25 Gew.-%ig)
1,3 l/h Wasser

Die Temperatur wurde im ersten Kessel auf 5-10 °C gehalten und betrug im zweiten Kessel 10-15 °C. Die pH-Werte betrugen in beiden Kesseln 10-11. Verweilzeit = Kessel 1-42 Min./Kessel 2-24 Min. Bei einer Fahrzeit von 9 Stunden wurde das Xanthogendisulfid in einer Ausbeute von 75-79 % erhalten.

Fp. = 51-53 °C
Freier Schwefel nach HPLC = 0,3 %.

Beispiel 8 (Vergleichsbeispiel)

Die Herstellung von Bis-(5-ethyl-1,3-dioxan-5-yl-methyl)-Xanthogendisulfid (MTX) erfolgt wie in DE-OS 2 06 610 beschrieben. Man erhält ein gelbliches Produkt mit einem Schmelzpunkt von 45-51 °C.

Beispiel 9 (Vergleichsbeispiel)

Das nach Beispiel 8 erhaltene Xanthogendisulfid wird wie folgt durch Umkristallisation gereinigt.

Das feuchte, noch nicht getrocknete MTX wird in Aceton aufgelöst und die Lösung filtriert. Es bildet sich eine MTX-Aceton- und eine $H_2O$-Salz-Phase. Durch zweimaliges Digerieren mit Wasser und nachfolgendem Abtrennen der Wasserphase, wird das Material von Wasser, Salz und Aceton befreit. Nun wird erneut mit Isopropanol und Aceton versetzt und auf ca. 30 °C erwärmt, damit alles MTX in Lösung geht. Danach wird auf 0 °C abgekühlt, das auskristallisierte MTX abgenutscht und getrocknet.

Man erhält ein fast weißes, feinkristallines Produkt mit einem Schmelzpunkt von 50-51 °C.

Beispiele 10-12

Das nach Beispiel 2A und $B_1$ sowie Beispielen 8 und 9 hergestellte MTW wird als Regler bei einer Chloroprenpolymerisation eigesetzt. Man polymerisiert wie folgt :

(M) = Monomerphase :

| | |
|---|---|
| Chloropren | 100,0 Gew.-Teile |
| MTX nach Beispiel 8, 9, 2A + $B_1$ | 0,7 Gew.-Teile |

(W) = wäßrige Phase :

| | |
|---|---|
| Entsalztes Wasser | 130,0 Gew.-Teile |
| Natriumsalz einer disproportionierten Abietinsäure | 4,0 Gew.-Teile |
| Natriumsalz eines Kondensationsproduktes aus Naphthalinsulfonsäure und Formaldehyd | 0,7 Gew.-Teile |
| Ätznatron | 0,63 Gew.-Teile |

(A) = Aktivatorphase :

2,5 Gew.-%ige wäßrige Formamidinsulfinsäurelösung

In einem 20 l-Reaktor werden die wäßrige und die Monomerphase gemischt und die entstandene Emulsion mit 5 Teilen der Aktivatorphase versetzt. Bei einer Innentemperatur von 40 °C springt die Reaktion leicht an. Durch eine Außenkühlung wird die freiwerdende Polymerisationswärme abgeführt und die Polymerisationstemperatur auf + 45 °C gehalten. Bei einem Monomerumsatz von 66 % wird die Reaktion durch Zugabe von Phenothiazin abgebrochen. Das restliche Monomere wird durch Wasserdampfdestillation aus dem Polymeren entfernt und der Polymerlatex nach Senken des pH-Wertes auf 7,0 auf einer Kühlwalze ausgefroren und isoliert. Die Mooney-Viskosität und der Aktivatorverbrauch sind der folgenden Tabelle zu entnehmen.

| Beispiel | MTX nach Beispiel | Aktivatorverbrauch (Gew.-Teile) | ML'-4 (ME) |
|---|---|---|---|
| 10 a) | 8 | 62 | 58 |
| 11 a) | 9 | 50 | 47 |
| 12 | 2 A + $B_1$ | 38 | 35 |

a) Vergleichsbeispiel

Beispiele 13-15

Die nach Beispiel 10-12 hergestellten Polymerlatices werden auf einer Kühlwalze ausgefroren und in einem Trockner, wie er in DE-OS 1 051 506 beschrieben worden ist, getrocknet. Der erfindungsgemäß hergestellte Kautschuk besitzt danach eine hellere Eigenfarbe. Zur Beurteilung der Farbe wurden die Polymeren in Toluol gelöt (20 Gew.-%), aus dieser Lösung 1 mm dicke Filme gegossen und nach dem Trocknen deren Farbe Musterkarten (RAL) des Ausschusses für Lieferbedingungen und Gütesicherung beim Deutschen Normenausschuß (DNA), 6 Frankfurt/M.1 Gutleutstr. 163-167, zugeordnet.

| Beispiel | Polymer nach Beispiel | Polymerfarbe nach RAL |
|---|---|---|
| 13 a) | 10 a) | 1015 |
| 14 a) | 11 a) | 1013 |
| 15 | 12 | 9001 |

a) Vergleichsbeispiel

Aus den Tabellen geht deutlich hervor, daß mit dem erfindungsgemäß hergestellten Regler eine wesentlich bessere Reglerwirksamkeit bei niedrigerem Aktivatorverbrauch erzielt wird (Beispiel 12) und ein Kautschuk mit heller Eigenfarbe herstellbar ist (Beispiel 15).

Beispiele 16-18

Für die Herstellung von Polychloropren-Klebstoff wurde eine Mischung folgender Zusammensetzung unter Stickstoffatmosphäre polymerisiert :

| | |
|---|---|
| Chloropren | 100,0 Gew.-Teile |
| MTX nach Beispielen 8, 9 oder 2A + $B_1$ | 0,48-0,61 Gew.-Teile |
| entsalztes Wasser | 140,0 Gew.-Teile |
| Natriumsalz einer disproportionierten Abietinsäure | 6,0 Gew.-Teile |
| Natriumsalz einer Naphthalinsulfonsäure/Formaldehyd-Kondensationsproduktes | 0,7 Gew.-Teile |
| Natronlauge (100 %) | 0,6 Gew.-Teile |
| Kaliumperoxodisulfat | 0,1 Gew.-Teile |

Die Polymerisation erfolgt bei + 10 °C bei kontinuierlichem Zulauf von 2 Gew.-%iger wäßriger Formamidinsulfinsäure. Bei einem Monomerumsatz von 71 % wird die Reaktion unter Zugabe von 0,1 Gew.-Teil Phenothiazin abgebrochen. Die Aufarbeitung erfolgt wie in Beispiel 10-12 beschrieben.

Beispiel 19

(Festigkeit der Klebung)

Durch Zusatz von Polyisocyanat wird die Abbindegegeschwindigkeit und die Sofortfestigkeit der Klebung verbessert. Ein solche Zweikomponentenklebstoff wird hergestellt, indem man das Polymere als 17 Gew.-%ige Lösung in Ethyl-acetat/Benzin 65-95 °C/Toluol im Gewichtsverhältnis 2 : 2 : 1 unter Rühren herstellt und mit 5 Teilen Triphenylmethan-4,4',4''-triisocyanat versetzt. Die Festigkeit der Klebung an einem NR-Vulkanisat wird nach DIN 53 273 ermittelt

| Beispiel | MTX nach Beispiel | MTX Menge Gew.-Teilen | ML-4 des Polymeren (ME) | Schälwiderstand der Klebung nach 10 h (N/mm) |
|---|---|---|---|---|
| 16 a) | 8 a) | 0,61 | 102 | 4 |
| 17 a) | 9 a) | 0,55 | 98 | 5,2 |
| 18 | 2 A + $B_1$ | 0,48 | 100 | 6,5 |

a) Vergleichsbeispiel

Wie man sieht, ist die Festigkeit der Klebung durch den erfindungsgemäß hergestellten Klebstoffrohstoff (Beispeil 18) deutlich besser.

Besipiel 20 (Vergleichsbeispiel)

Man stellt das Xanthogendisulfid des 3-Methoxybutanol-1 nach DE-OS 2 156 453 her und polymerisiert Chloropren in Gegenwart von 0,65 Gew.-Teilen wie in Beispielen 10-12 beschrieben. Man erhält nach der Aufarbeitung einen Festkautschuk mit einer Mooney-Viskosität von ML-4' = 54 ME.

Beispiel 21

Man verfährt wie im Beispiel 21 beschrieben, nur stellt man das Xanthogenat unter erfindungsgemäßen Reaktionsbedingungen her, wie im Beispiel 2A und $B_1$ beschrieben.

Der Kautschuk hat eine Mooney-Viskosität von ML-4' = 38 ME.

**Ansprüche**

1. Verfahren zur Herstellung Xanthogendisulfiden der Formel

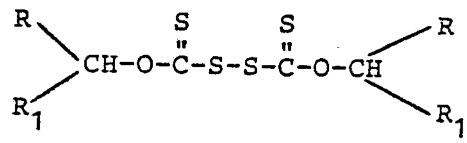

in der

R und $R_1$ unabhängig voneinander Wasserstoff, $C_1$-$C_6$-Alkyl, $C_5$-$C_8$-Cycloalkyl oder ein sauerstoffhaltiges, gegebenenfalls 1- bis 3-fach durch $C_1$-$C_4$-Alkyl substituiertes, heterocyclisches Ringsystem mit 5-8 Ringgliedern darstellen, wobei

R und $R_1$ darüber hinaus auch miteinander zu einem heterocyclischen Ring mit 3-6 C-Atomen und 1-3 Sauerstoffringgliedern verbunden sein können, der gegebenenfalls durch 1-3 $C_1$-$C_4$-Alkylgruppen substituiert sein kann, durch Umsetzung eines Xanthogensäure-O-ester-alkalisalzes der Formel

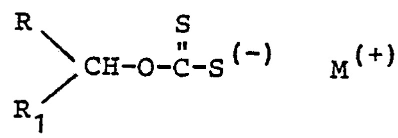

in der

R und $R_1$ die vorstehend genannte Bedeutung besitzen und

M für ein Alkalimetallatom steht,

mit wäßriger Chlorbleichlauge, bei einer Temperatur von − 10 bis 20 °C und bei einem pH-Wert von 8 bis 12, dadurch gekennzeichnet, daß man wäßrige Chlorbleichlauge einer Konzentration von weniger als 0,9 Mol NaOCl/kg einsetzt und nur soweit oxidiert, daß zum Ende der Reaktion das Xanthogensäure-O-ester-alkalisalz noch zu 0,1 bis 5,0 Mol-%, bezogen auf die eingesetzte Menge, vorhanden ist.

2. Verfahren gemäß Anspruch 1, dadurch gekennzeichnet, daß man wäßrige Chlorbleichlauge einer Konzentration von 0,4 bis 0,7 Mol/kg einsetzt.

3. Verfahren gemäß Anspruch 1, dadurch gekennzeichnet, daß man nur soweit oxidiert, daß zum Ende der Reaktion das eingesetzte Xanthogensäure-O-ester-alkalisalz noch zu 1 bis 3 Mol-%, bezogen auf die eingesetzte Menge, vorhanden ist.

4. Verwendung der gemäß Anspruch 1 erhaltenen Xanthogendisulfide als Molgewichtsregler bei der Polymerisation von Chloropren.

**Claims**

1. Process for the preparation of xanthogen disulphides of the formula

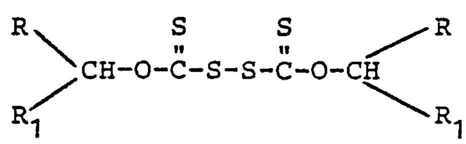

in which

R and $R_1$ independently of one another represent hydrogen, $C_1$-$C_6$-alkyl, $C_5$-$C_8$-cycloalkyl or an oxygen-containing heterocyclic ring system which has 5 to 8 ring members and is optionally substituted 1 to 3 times by $C_1$-$C_4$-alkyl,

it further being possible for R and $R_1$ to be connected to one another to complete a heterocyclic ring with 3-6 C atoms and 1-3 oxygen ring members, which heterocyclic ring can optionally substituted by 1-3 $C_1$-$C_4$-alkyl groups, by reacting a xanthic acid-O-ester-alkali metal salt of the formula

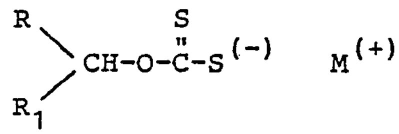

in which

R and $R_1$ have the aforementioned meaning and

M represents an alkali metal atom,

with aqueous chlorine bleach liquor, at a temperature of − 10 to 20 °C and at a pH value of 8 to 12, characterised in that aqueous chlorine bleach liquor having a concentration of less than 0.9 mol NaOCl/kg is used and oxidation is carried out only until 0.1 to 5.0 mol % of the xanthic acid-O-ester-alkali metal salt, based on the quantity used, is still present at the end of the reaction.

2. Process according to Claim 1, characterised in that aqueous chlorine bleach liquor having a concentration of 0.4 to 0.7 mol/kg is used.

3. Process according to claim 1, characterised in that oxidation is carried out only until 1 to 3 mol % of the xanthic acid-O-ester-alkali metal salt used, based on the quantity used, is still present at the end of the reaction.

4. Use of the xanthogen disulphides obtained according to Claim 1 as molecular weight regulators in the polymerisation of chloroprene.

**Revendications**

1. Procédé de fabrication de disulfure de xanthogène de formule

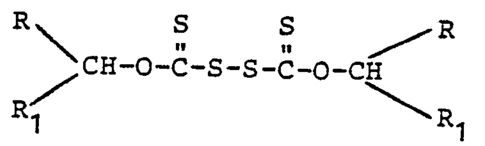

dans laquelle

R et $R_1$ représentent indépendamment l'un de l'autre de l'hydrogène, un alcoyle en $C_1$-$C_6$, un cycloalcoyle en $C_5$-$C_8$ ou un système nucléaire hétérocyclique contenant de l'oxygène, éventuellement 1 à 3 fois substitué par un alcoyle en $C_1$-$C_4$ et ayant 5 à 8 chaînons nucléaires,

R et $R_1$ pouvant en outre aussi être reliés entre eux en un noyau hétérocyclique ayant 3 à 6 atomes de carbone et 1 à 3 chaînons nucléaires oxygène, qui peut être substitué éventuellement par 1 à 3 groupes alcoyle en $C_1$-$C_4$, par réaction d'un sel alcalin d'un O-ester d'acide xanthogénique de formule

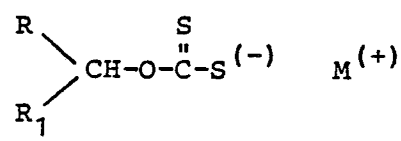

dans laquelle

R et $R_1$ possèdent la signification citée plus haut et

M représente un atome de métal alcalin, avec une liqueur aqueuse de blanchiment au chlore, à une température de − 10 à 20 °C et à une valeur de pH de 8 à 12, caractérisé en ce qu'on utilise une liqueur aqueuse de blanchiment au chlore ayant une concentration inférieure à 0,9 mole de NaOCl/kg et en ce qu'on oxyde seulement suffisamment pour qu'à la fin de la réaction xanthogénique soit encore présent à raison de 0,1 à 0,5 moles %, par rapport à la quantité mise en jeu.

2. Procédé selon la revendication 1, caractérisé en ce qu'on utilise une solution aqueuse de blanchiment au chlore ayant une concentration de 0,4 à 0,7 mole/kg.

3. Procédé selon la revendication 1, caractérisé en ce qu'on oxyde seulement suffisamment pour qu'à la fin de la réaction le sel alcalin de l'O-ester d'acide xanthogénique employé soit encore présent à raison de 1 à 3 moles %, par rapport à la quantité mise en jeu.

4. Utilisation des disulfures de xanthogène obtenus selon la revendication 1 comme régulateurs du poids moléculaire dans la polymérisation du chloroprène.

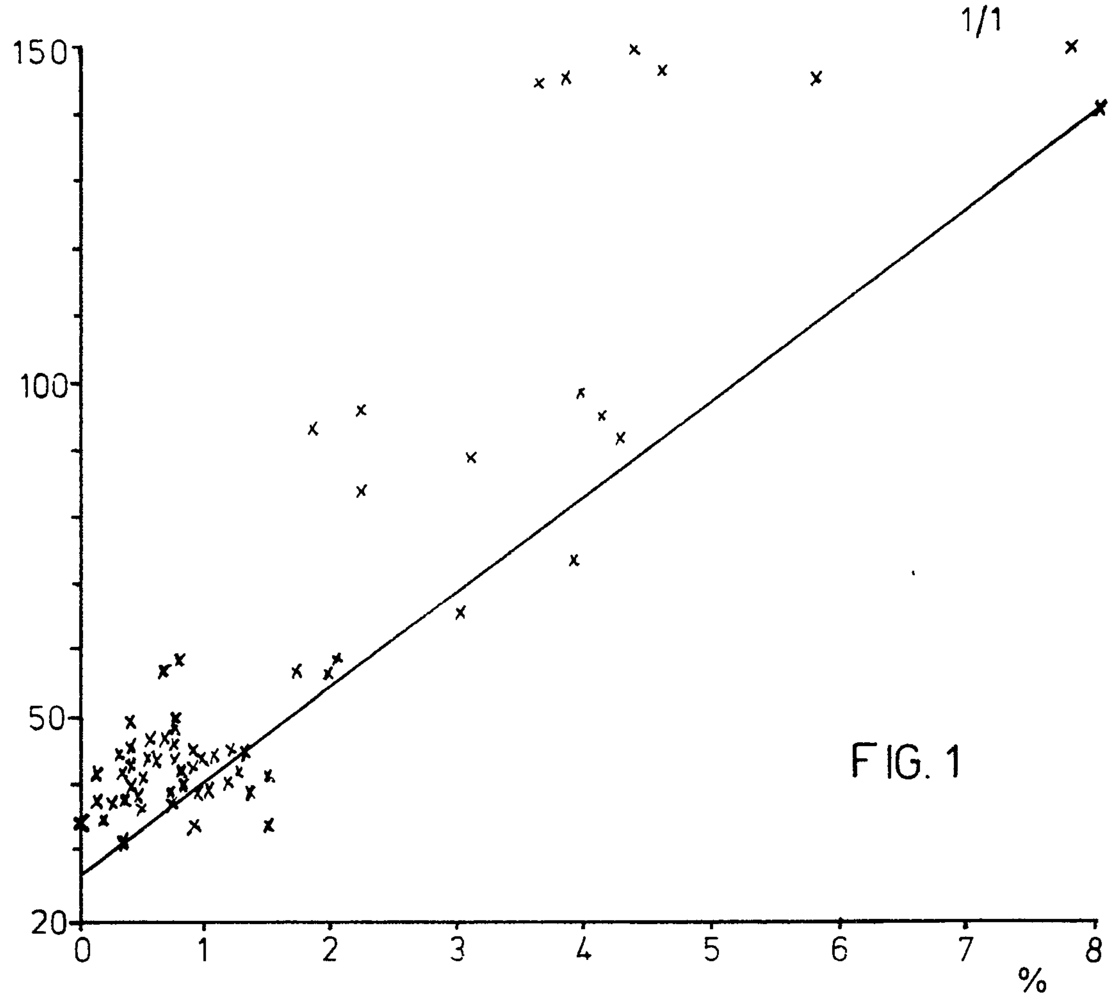


FIG. 1

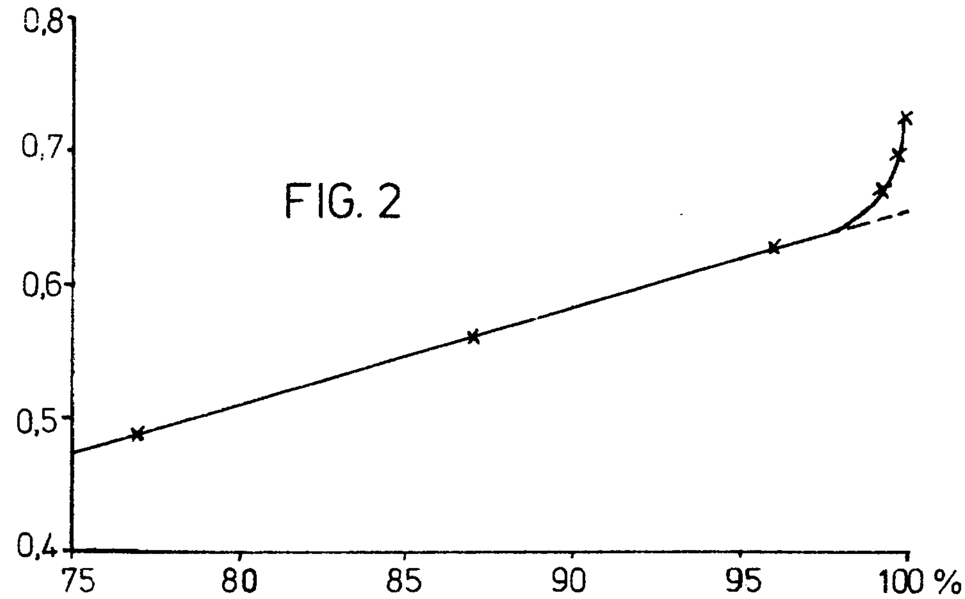


FIG. 2